# EUROPEAN PATENT APPLICATION

(11) **EP 1 829 976 A1**
(43) Date of publication of application: **05.09.2007**
(21) Application number: 05805120.2
(22) Date of filing: 24.10.2005
(51) Int. Cl.: C12Q 1/06, C12M 1/34, G01N 21/64

(54) **METHOD OF EVALUATING CELLS, SYSTEM FOR MEASURING CELLS AND PROGRAM FOR MEASURING CELLS**

(30) Priority: 08.12.2004 JP 2004355235
(71) Applicant: OSAKA UNIVERSITY, Suita-shi, Osaka 565-0871 (JP)
(72) Inventor: MATSUNAGA, Sachihiro, (JP); UCHIYAMA, Susumu, (JP); FUKUI, Kiichi, (JP)
(74) Representative: Jacob, Reuben Ellis
(86) International application number: PCT/JP2005/019517
(87) International publication number: WO 2006/061958

(57) **Abstract**

A method for monitoring cells is provided capable of determining the stage in cell cycle of a cell in a rapid and highly reliable manner.
A step of obtaining an image that reflects the chromosomal state in the cell, and a step of calculating a parameter that corresponds to the chromosomal state based on the image to determine the stage in the cell cycle on the basis of the calculation result are included.

## Description

### TECHNICAL FIELD

The present invention relates to a method for monitoring cells, a system for cell-based assay, and a program for cell-based assay.

### BACKGROUND ART

Evaluation of a cell activity is useful not only in elucidation of cellular biological, biochemical events and properties but also as measures for examining bioactivities and toxicity of chemical substances. Hence, procedures for the evaluation of activities have been investigated.

Examples of common methods for evaluating a cell activity include: a method in which the amount of a dye such as trypan blue or neutral red incorporated into cells is assayed with an absorptiometer, and the cell activity is evaluated based on the assay results; a method in which the quantity of an enzyme such as dehydrogenase leaked outside of the cells is determined based on the activity, and the cell activity is evaluated based on the determination results; and a method in which an electrode is inserted in a cell suspension, and an electric current value derived from cellular electric activity is assayed through applying a certain potential to the electrode, whereby the cell activity is evaluated based on the assay results; and the like.

However, the cell activity is indirectly assayed and the cell activity is evaluated based on the assay results in these methods, therefore, the detection was difficult when the cell activity varies slightly. Moreover, it was difficult to reveal the relationship between cell activity and cellular biological information on e.g., the most affected stage in the cell cycle, and the like.

As methods for solving such problems, methods in which morphological alteration of cells or intracellular organelles is observed can be illustrated. Cell morphology is altered during the cell division. In this event, state of condensation of the chromosome that is an intracellular organelle is greatly changed in particular, thereby causing chromosomal separation and partition. It is known that the chromosomal separation and partition is essential for cell division, and the alteration of the cell activity affects the chromosome separation in mitotic phase. Therefore, evaluation of the cell activity is enabled by the results of observation of features of morphological alteration of chromosomes. As a method of observation of the morphological alteration of chromosomes, a method in which the chromosome is visualized using a fluorescent dye, and the morphological alteration of the chromosome is tracked has been known (see, Patent Document 1).
Patent Document 1: JP-A No. H8-136536

### DISCLOSURE OF THE INVENTION

### Problems that the Invention is to Solve

The method according to Patent Document 1 determines the pharmaceutical effect by counting cell number in the cell division, and comparing percentage ratio to the total number with that of a control group. In this step, the fluorescence image of chromosomes is used to determine as to whether or not the cell in the mitotic phase. However, the determination is made by visual observation, therefore, reliability is insufficient, and rapid determination may be difficult. In Example 1 of Patent Document 1, mitotic stage of dividing cells is also determined, however, determination of further detailed stages by visual observation is difficult, suggesting lack in reliability.

Additionally, in the method of Patent Document 1, chromosome is visualized by staining DNAs that are constitutive components of the chromosome using 4',6-diamidino-2-phenylindole dihydrochloride as a fluorescent dye, however, use of this fluorescent dye at a concentration commonly employed leads to cell death. After the cell death, chromosomal morphology in the dead cell can be observed in use of the fluorescent dye, however, chromosomal morphological alteration in living cells cannot be observed continuously. Furthermore, fluorescence intensity of the chromosomes stained using the fluorescent dye is reduced by half every cell division to reach to the fluorescence intensity below the detection limit, therefore, continuous observation for a long period of time is not possible. Moreover, in the method of Patent Document 1, there is the description that a pharmaceutical effect of anticancer agents can be detected by tracking the morphological alteration in mitotic phase of cells. However, the pharmaceutical effect is determined by counting cell number in the cell division, and comparing percentage ratio in the whole cells with that in control group. Therefore, the method would merely detect great effects which may influence on the entire mitotic phase taking into consideration of proportion of the cells in the mitotic phase generally accounts for 4% or lower in the whole cells. Accordingly, the detection would be difficult when the influence is a little such as in poison tests, environmental endocrine disrupter tests and the like.

### Means for Solving the Problems

An object of the present invention in order to solve the foregoing problems is to provide a method capable of determining the stage in cell cycle in a rapid and highly reliable manner. Moreover, another object of the present invention is to provide a method capable of determining the stage in the living state, without killing the cell. Further, still another object of the invention is to provide a system for cell-based assay which automatically determines the cell stage, and a program for cell-based assay to allow a computer to execute the determination of the cell stage.

One aspect of the present invention is a method for monitoring a cell which includes: (a) obtaining an image that reflects the chromosomal state in the cell; and (b) calculating a parameter that corresponds to the chromosomal state based on the image to determine the stage in the cell cycle on the basis of the calculation result.

The parameter may include, preferably, chromosomal circularity, chromosomal roundness, ratio of lengths of chromosomal major axis and minor axis, chromosomal eccentricity, chromosomal Feret's diameter, distance between the centroids of a chromosome and a chromosome adjacent thereto, angle formed by a chromosomal major axis and the major axis of a nuclear chromosome adjacent thereto, or any combination of these. The term chromosome referred to herein means a group of chromosomes unless otherwise stated. Moreover, the group of chromosomes in the interphase of cell division is also referred to as nucleus, and the chromosome referred to herein also includes the same.

In one preferred mode of the aforementioned method for monitoring a cell, a step (c) of allowing a fusion protein of a chromosomal protein and a protein that generates fluorescence to be expressed in the cell is included before the step (a). In this case, the image obtained in the step (a) is a fluorescence image originating from the fluorescence generated by the cell. According to this method, the fluorescence image derived from the chromosomal state can be obtained without killing the cell.

In the aforementioned method for monitoring a cell, a step (d) of evaluating the degree of activity of the cell on the basis of the result of determination in the step (b) may be further included.

In one mode of the method for monitoring a cell: images that correspond to multiple cells in a sample are obtained in the step (a); the stages are determined respectively on the multiple cells in the step (b); and the proportion of cell number in each stage is calculated in the step (d), whereby the degree of activity of the cell is evaluated on the basis of the calculation result.

Additionally, in one mode of the method for monitoring a cell, the images that correspond to a particular cell are obtained at certain time intervals in the step (a); the stage at certain time intervals of the particular cell is determined in the step (b); and the degree of activity of the cell is evaluated on the basis of the information of alteration of the stage of the particular cell with respect to time course in the step (d).

Furthermore, another aspect of the present invention is a system for cell-based assay which includes: a fluorescence detection apparatus for detecting fluorescence that reflects the chromosomal state in a cell; an imaging apparatus for obtaining a fluorescence image originating from fluorescence detected by the fluorescence detection apparatus; and an image analysis apparatus for calculating a parameter that corresponds to the chromosomal state based on the fluorescence image obtained by the imaging apparatus, and determining the stage in the cell cycle on the basis of the calculation result.

In one mode of the system for cell-based assay, the fluorescence detection apparatus simultaneously detects fluorescence derived from multiple cells in a sample; the imaging apparatus obtains the fluorescence image involving the fluorescent information generated by the multiple cells in the sample; and the image analysis apparatus extracts the fluorescence image that corresponds to each cell from the fluorescence image obtained by the imaging apparatus to determine the stage in cell cycle on each cell.

In one mode of the system for cell-based assay, the imaging apparatus obtains the fluorescence image that corresponds to a particular cell at certain time intervals, and the image analysis apparatus determines the stage in the cell cycle at certain time intervals of the particular cell.

Also, yet another aspect of the present invention is a program for cell-based assay wherein: fluorescence that reflects the chromosomal state in a cell and that is generated by the cell is detected; a fluorescence image is obtained from the fluorescence; a parameter that corresponds to the chromosomal state is calculated based on the fluorescence image; and a computer is allowed to execute procedures for determining the stage in the cell cycle on the basis of the calculation result. Still further, other aspect of the invention is a computer readable recording medium in which the program for cell-based assay is recorded.

### Advantages of the Invention

According to the present invention, rapid, convenient and highly reliable monitoring can be carried out because cell monitoring is conducted on the basis of the parameter that corresponds to the chromosomal state. Furthermore, because the information on the stage in mitotic phase of the cell can be obtained, detection of an alteration is enabled which is so slight that the entire mitotic phase may not be influenced. Similarly, monitoring of influences of an inhibitory substance on the cell activity is permitted even at a low concentration.

According to the aspect of the invention of Claim 3, the fluorescence image of the cell can be obtained without introducing a compound, which can affect the cell life, from the exterior of the cell. In other words, the activity of not dead cells but living cells can be evaluated.

Furthermore, according to the system for cell-based assay and the program for cell-based assay of the present invention, cell cycle stage can be conveniently determined on living cells, and the determination results are useful in evaluating the degree of activity of the cell.

### BRIEF DESCRIPTION OF THE DRAWINGS

[Fig. 1] Fig. 1 shows a drawing illustrating fluorescence microscope images in each stage in the cell cycle of HeLa cells.
[Fig. 2] Fig. 2 shows a block diagram illustrating an electrical configuration of the system for cell-based assay in Examples.
[Fig. 3] Fig. 3 shows a schematic view illustrating one example of construction in appearance of the system for cell-based assay in Examples.
[Fig. 4] Fig. 4 shows a view illustrating processing, procedures in the system for cell-based assay in Examples.
[Fig. 5] Fig. 5 shows a view illustrating processing procedures in determination of the stage in the system for cell-based assay in Examples.
[Fig. 6] Fig. 6 shows one example of a display of the determination results of the stage in Examples.
[Fig. 7] Fig. 7 shows a drawing illustrating other processing procedures in determination of the stage in the system for cell-based assay in Examples.
[Fig. 8] Fig. 8 shows a drawing illustrating the results of time-lapse analysis in an experiment.

### Description of Reference Numerals and Signs

1, 1a: system for cell-based assay
2: fluorescence detection apparatus
2a: fluorescence microscope
3: imaging apparatus
3a: camcorder
4: image analysis apparatus
4a: personal computer
5: output device
5a: monitor
6: input device
6a: keyboard
6b: mouse
7: auxiliary memory device
8: program memory medium
41: control unit
42: memory unit

### BEST MODE FOR CARRYING OUT THE INVENTION

Hereinafter, modes for carrying out the present invention will be described with reference to the drawings.

The method for monitoring a cell of the present invention is a method which includes obtaining an image that reflects the chromosomal state in the cell, and calculating a parameter that corresponds to the chromosomal state based on the image to determine the stage in the cell cycle on the basis of the calculation result. On the basis of such monitoring, a cell activity can be evaluated.

As the process for obtaining the image that reflects the chromosomal state in the cell, various known processes can be employed. One example of preferable processes may be a process in which a fusion protein containing a chromosomal protein and a protein that generates fluorescence is allowed to be expressed, and the fluorescence image is obtained from the fluorescence generated by the cell. According to this method, living cells can be readily the monitoring subject without need of introducing a compound which can be toxic to cells from the exterior of the cell. Hereinafter, a case in which this process is employed will be explained.

As the monitoring subject cell, any cells which are not killed even though a fluorescence protein is introduced therein, exhibit a mitotic activity, and include the fusion protein normally located on the chromosome are acceptable. The method according to the present invention can be used in monitoring of, for example, HeLa cells being human cervical cancer cells, CHO cells being Chinese Hamster Ovary fibroblast, BY-2 cells being tobacco suspension culture cells, Hep cells being cells derived from human pharyngeal cancer, and the like.

The aforementioned chromosomal protein may be any chromosomal protein which exhibits localization in the chromosome in cell division, and the localization of which is not altered by addition of a fluorescence protein. Examples of the chromosomal protein which may be preferably used include e.g., histone, condensin and topoisomerase.

Examples of the aforementioned fluorescence protein include green fluorescence protein (GFP), yellow fluorescence protein (YFP), red fluorescence protein (dsRed), and modified products thereof, and the like.

As the process for constructing the cell that expresses a fusion protein of the chromosomal protein and the fluorescence protein may be any known process.

Because chromosomes are composed of DNAs and chromosomal proteins, information about the degree of condensation and morphological alteration of the chromosome can be obtained from the fluorescence image of the DNAs. On the other hand, although direct observation of the chromosomal protein is difficult, the information about the degree of condensation and morphological alteration of the chromosome can be obtained from the fluorescence image of the fluorescence protein fused to the chromosomal protein as in this embodiment similarly to the fluorescence image of the DNAs. The followings are Experimental Examples showing this matter.

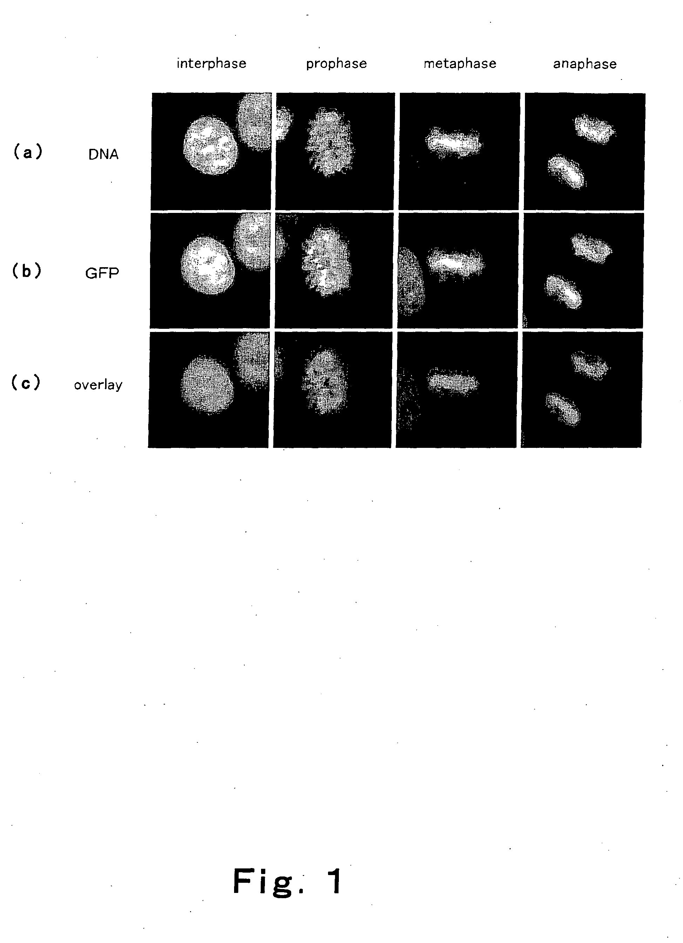

Fig. 1 shows a drawing illustrating fluorescence microscope images in each stage in the cell cycle of HeLa cells. Herein, cell cycle is divided into interphase and mitotic phase; and the mitotic phase is further divided into stages of prophase, prometaphase, metaphase, anaphase and telophase. In Fig. 1, fluorescence microscope images in the interphase, prometaphase, metaphase and anaphase are shown. In the experiment for obtaining the fluorescence, microscope images shown in Fig. 1, a GFP-histone H1 fusion protein was expressed in the cell, and stained the DNAs in the cell with Hoechst (Hoechst 33342). Fig. 1 (a) shows a fluorescence microscope image obtained by exciting Hoechst followed by passing through a filter corresponding to the fluorescence wavelength of Hoechst, i.e., a fluorescence microscope image of the DNA. Fig. 1 (b) shows a fluorescence microscope image obtained by exciting GFP followed by passing through a filter corresponding to the fluorescence wavelength of the GFP. Fig. 1 (c) shows a fluorescence microscope image obtained by overlaying the fluorescence microscope image shown in Fig. 1 (a), and the fluorescence microscope image shown in Fig. 1 (b). It is revealed that the fluorescence microscope image shown in Fig. 1 (a) almost matches the fluorescence microscope image shown in Fig. 1 (b). Accordingly, because the chromosome is composed of DNAs and chromosomal proteins, the degree of the condensation and the morphological alteration of the chromosome can be found by obtaining the fluorescence microscope images of the DNAs, while the degree of the condensation and the morphological alteration of the chromosome can be also found from the fluorescence microscope image of the GFP.

The information about the degree of the condensation and the morphological alteration of the chromosome is useful in determining the stage in the cell cycle. The present inventor considered that determination of the stage in cell cycle can be carried out in a convenient, rapid and accurate manner by obtaining the information about the chromosomal state such as the degree of the condensation and the morphological alteration of the chromosome through digitalization. Hence, a variety of parameters that may correspond to the chromosomal state were searched, and parameters that are useful in determination of the stage in cell division were investigated. As a result of the investigation, it was found that chromosomal circularity, chromosomal roundness, distance between the centroids of a chromosome and a chromosome adjacent thereto, angle formed by a chromosomal major axis and the major axis of a chromosome adjacent thereto, ratio of lengths of chromosomal major axis and minor axis, chromosomal eccentricity, and chromosomal Feret's diameter can be utilized as efficacious parameters. Moreover, it was proven that more detailed information about the chromosomal state can be obtained by using a combination through selecting these ad libitum as a determination standard.

In the following Examples, the stage in the cell cycle is determined by using multiple parameters as the determination standard among the aforementioned ones. However, the present invention is not limited to the embodiment in which determination is made on the basis of these parameters. In this embodiment, when each parameter of the chromosome is calculated, shape of the periphery provided by the contour of the chromosomes is defined as the shape corresponding to the chromosome. Accordingly, chromosomal circularity, roundness and the like are defined as the circularity, roundness and the like of shape of the periphery provided by the contour of the chromosomes.

The chromosomal circularity referred to herein means a value derived by dividing a value of (area x 4π) by a square value of the length of the line enclosing the chromosomal area. The chromosomal roundness means a value derived by dividing a value of π x (the length of the major axis)^{0.5} by a value of (4 x area). The adjacent chromosome refers to a chromosome the centroid of which attains the minimum distance from the centroid of the subject chromosome. The angle formed by a chromosomal major axis and the major axis of a chromosome adjacent thereto means a minimum angle between major axes calculated assuming ellipses that are most fitted to the shape of each chromosome. The ratio of the major axis to the minor axis of the chromosome means a ratio of the major axis to the minor axis calculated assuming an ellipse that is most fitted to the shape of the chromosome. The chromosomal eccentricity is a value suggesting the deviation from an ideal perfect circle, and means, in the case of the chromosome being sandwiched with two concentric circles, the difference between the maximum radius and the minimum radius measured with respect to the center when minimum difference between the radii of the concentric circles are yielded. The chromosomal Feret's diameter means the length, when two points are envisaged on the periphery of the shape of the chromosome, to yield the maximum distance between the two points.

Next, specific examples of the method according to the present invention for evaluating the degree of activity of the cell on the basis of the determination results of the stage in the cell cycle will be explained. For example, a method including using multiple cells included in a single sample as a subject to determine the stage in the cell cycle on each cell, and deciding the proportion of cell number in each stage to total cell number (hereinafter, may be also referred to as "count mode"), whereby the degree of activity of the cell is evaluated on the basis of the proportion; and a method including detecting the state of transition of the stage depending on time course on particular cell (may be either a single cell or multiple cells) (hereinafter, may be also referred to as "time-lapse mode"), whereby the degree of activity of the cell is evaluated on the basis of the detection result may be illustrated. A plurality of methods may be employed in combination. Any one of these methods evaluates the degree of activity of the cell by comparing with control data. For example, a case in which the method for evaluating a cell activity according to the present invention is applied for detecting responsiveness of a cell to a reagent will be explained. In the case according to the count mode, the proportion of cell number in each stage to total cell number is decided with respect to the cell which was brought into contact with a reagent; a graph of cellular distribution based on the proportion is produced; and the graph is compared with a graph of cellular distribution with respect to the cell which was not brought into contact with the reagent. When there arises a significant difference between the graphs of the cellular distribution, it is revealed that the cell is affected by the reagent. For example, when the proportion of the cell in interphase is increased while the proportion of the cell in mitotic phase is decreased, lowering of mitotic activity of the cell is proven.

The determination step of the stage in the cell cycle described above, and the subsequent evaluation step of the degree of activity of the cell may be carried out either with an apparatus for automatic execution, or with visual inspection by an observer. In the following Examples, a case in which the determination of the stage in the cell cycle is carried out by an automatic system for cell-based assay, and the subsequent evaluation step of the degree of activity of the cell is carried out with visual inspection by an observer will be demonstrated.

### Examples

### Automatic System for Cell-Based Assay

The system for cell-based assay according to the present invention will be explained below.

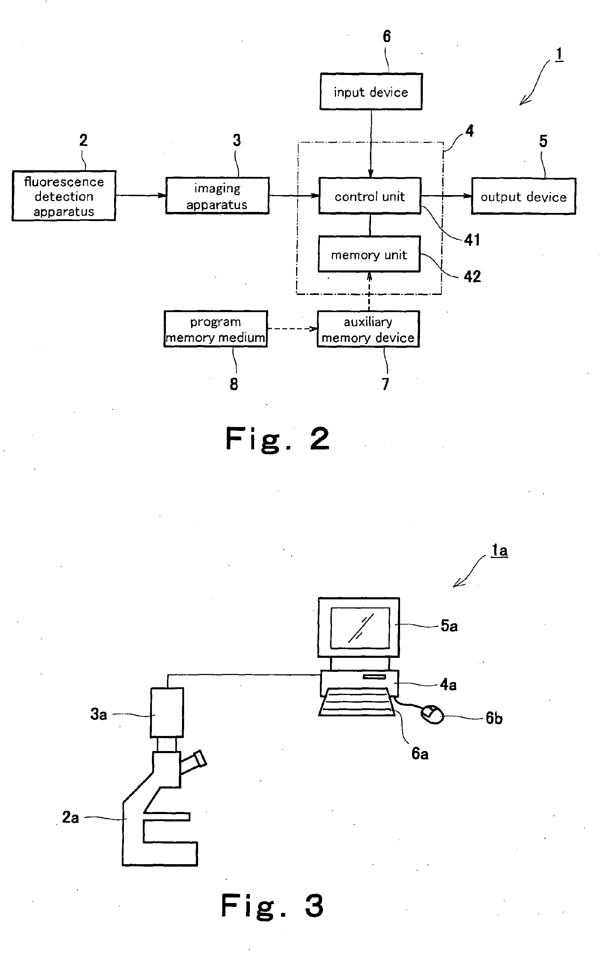

Fig. 2 shows a block diagram illustrating an electrical configuration of the system for cell-based assay 1 in Examples. The system for cell-based assay 1 in this Example includes a fluorescence detection apparatus 2 for detecting fluorescence, an imaging apparatus 3 for taking the fluorescence image originating from the fluorescence detected by the fluorescence detection apparatus 2, an image analysis apparatus 4 for analyzing the fluorescence image taken by the imaging apparatus 3, an output device 5 for output of the analytical results in the image analysis apparatus 4, and an input device 6 for carrying out various types of operations. The image analysis apparatus 4 includes a control unit 41 and a memory unit 42. The memory unit 42 stores a program for determining the cell stage that executes the processing described later, and the control unit 41 executes image analysis and the like according to the program and controls each apparatus.

Fig. 3 shows a schematic view illustrating one example of construction in appearance of the system for cell-based assay shown in Fig. 2. The system for cell-based assay 1a shown in Fig. 3 includes a fluorescence microscope 2a for detecting fluorescence, a camcorder 3a for imaging the fluorescence detected by the fluorescence microscope 2a as a fluorescence image, a personal computer (hereinafter, referred to as PC) 4a for analyzing the fluorescence image taken by the camcorder 3a, a monitor 5a for the output of the analytical results by PC 4a, a keyboard 6a and a mouse 6b for carrying out various types of operations. Upon determination of the stage of the cell cycle, a sample is placed on a mounting platform of the fluorescence microscope 2a.

The fluorescence detection apparatus 2 in the system for cell-based assay 1 may be any apparatus which can detect intensity of the fluorescence emitted from the sample, and a fluorescence microscope 2a is preferably used. Alternatively, other fluorescence scanner can be also used. As the imaging apparatus 3, for example, a camcorder 3a that takes the image signal from the fluorescence detection apparatus 2 as a secondary monochrome image can be used. The image analysis apparatus 4 can be constructed with an image processing circuit which can subject the imaging window at certain time intervals to processing at real time, and a microcomputer composed of CPU, ROM, RAM and I/O port. The image analysis apparatus 4 can be also constructed with, for example, PC 4a. The memory unit 42 of the image analysis apparatus 4 is constructed with ROM or RAM, and stores a program for analysis, however, the program for analysis stored on a program-recording medium 8 such as a floppy (registered trademark) disk, CD-ROM or the like may be read on a memory unit 42 via an auxiliary memory device 7 that is a mechanically readable/writable apparatus (floppy (registered trademark) disk drive, CD-ROM drive or the like) for the program-recording medium 8. As the output device 5, for example, a monitor 5a for the window such as CRT or a liquid crystal display, a printing apparatus for printing on paper such as a laser printer can be used. As the input device 6, a keyboard 6a, a mouse 6b and the like can be used.

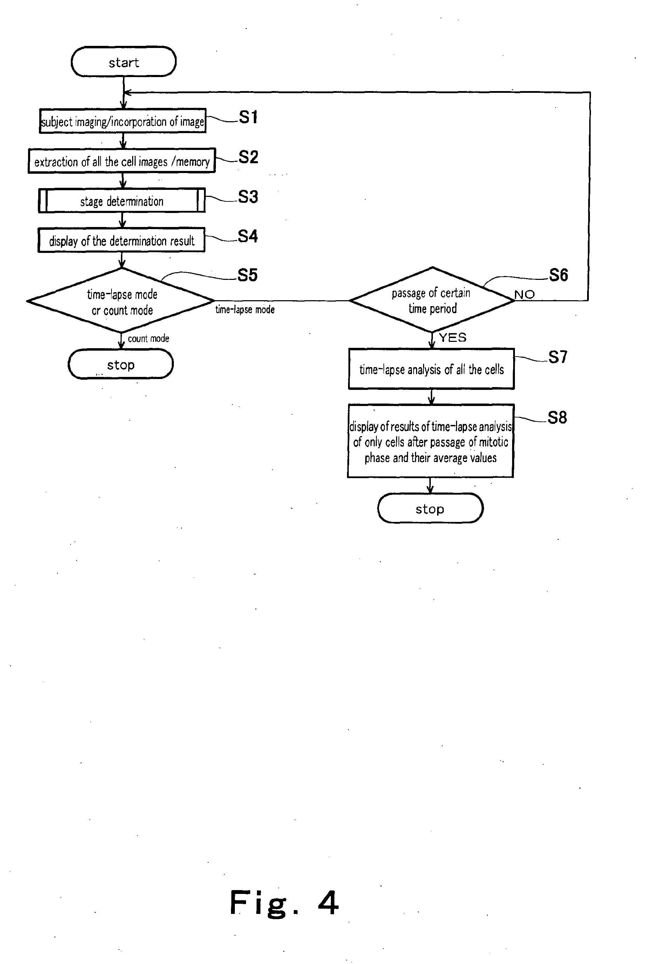

Fig. 4 shows a view illustrating processing procedures in the system for cell-based assay 1. Prior to initiation of the control in the system for cell-based assay 1, the operator selects either the time-lapse mode or the count mode using an input device 6. When the time-lapse mode is selected, tracking time is entered. Furthermore, the sample is set in the fluorescence detection apparatus 2. First, in step S1, the fluorescence image originating from the fluorescence detected by the fluorescence detection apparatus 2 is taken, and this fluorescence image is incorporated as image data. In the subsequent step S2, the cell image is extracted from the image data, and the extracted cell image is stored at the image memory of the memory unit 42 in the image analysis apparatus 4. Although one sample includes multiple cells, in general, ID Number is assigned on each cell from No. 1 in sequence, and the extracted each cell image is stored at the image memory so as to match to each ID Number in this step S2. In this step, decision of chromosomal centroid of each call is also carried out, and the position of the centroid is stored at the image memory so as to match to the ID Number. With respect to the cells in the anaphase and telophase of the cell division, two chromosomal images shall be present within one cell, however, in this case, the centroid matched to each chromosomal image is stored on the decision image memory. When the distance between the centroids of the two chromosomal images is smaller than a certain value, the two chromosomal images may be subjected to the processing in which they are derived from one cell, and the extraction of cell image may be conducted on the basis of such decision. Next, in step S3, determination of the stage of each cell is carried out.

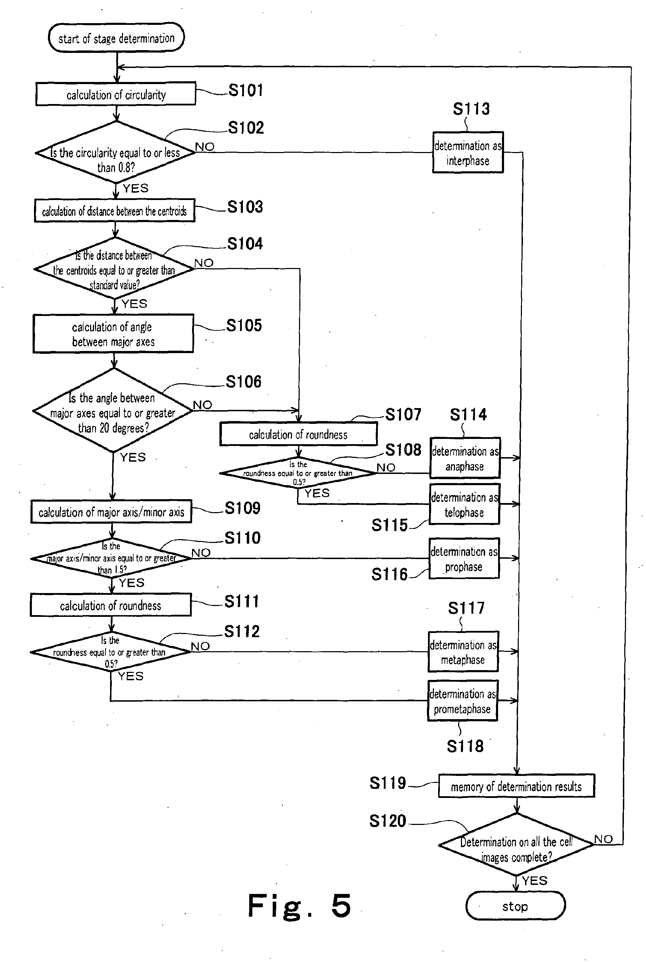

Fig. 5 shows a view illustrating specific processing procedures in determination of the stage of each cell in the step S3. Fig. 5 shows processing procedures in the case of cells that are expressing a GFP-histone H1 fusion protein. Preferred embodiment of the system for cell-based assay 1 may be constructed such that a value to be the determination standard on each parameter is selected depending on the cell type. As shown in Fig. 5, when the stage determination is started, the cell of ID No. 1 is first subjected to the following processing based on the cell image of ID No. 1.

Circularity is first calculated in step S101. Then, in the step S102, determination is made as to whether the circularity is equal to or less than 0.8. If not (NO), the stage of the cell is determined as the interphase in step S112. Although a value of 0.8 was employed as the standard value of the circularity in the step S101, a value falling within the range of 0.60 to 0.99 may be preferably employed. When the circularity is equal to or less than 0.8 (YES), the distance between the centroids of a chromosome and a chromosome adjacent thereto is calculated in step S103. The adjacent chromosome may be either the chromosome within single cell, or the chromosome of a different cell. Then, in step S104, determination is made as to whether the distance between the centroids calculated in the step S103 is equal to or greater than a standard value. When the distance is not equal to or greater than the standard value (NO) in the step S104, the next step S107 is executed. The standard value in the step S104 may be decided on every cell used. For example, when a HeLa cell is used, the standard value which can be used falls within the range of preferably 15 to 21 µm, and is particularly preferably 18 µm; when a CHO cell is used, the standard value which can be used falls within the range of preferably 14 to 20 µm, and is particularly preferably 17 µm; and when a BY-2 cell is used, the standard value which can be used falls within the range of preferably 17 to 23 µm, and is particularly preferably 20 µm. On the other hand, when the distance between the centroids calculated in the step S103 is equal to or greater than the standard value (YES), the angle formed by a chromosomal major axis and the major axis of a chromosome adjacent thereto is calculated in the step S105.

The angle calculated in S105 is determined in step S106 as to whether it is equal to or greater than 20 degrees. If not (NO), step S107 is executed. To the contrary, when the angle measured in the step S105 is equal to or greater than 20 degrees (YES), step S109 is executed. In the step S107, chromosomal roundness is calculated. In step S108, the roundness is determined as to whether it is equal to or greater than 0.5. When the roundness is equal to or greater than 0.5 (YES), the stage of the cell is determined as telophase in step S115. When the roundness is not equal to or greater than 0.5 (NO), the stage of the cell is determined as anaphase in the step S114.

In the step S109, the ratio of the major axis to the minor axis of the chromosome (major axis/minor axis) is calculated, and the major axis/minor axis is determined in the step S110 as to whether it is equal to or greater than 1.5. If not (NO), the stage of the cell is determined as prophase in the step S116. When the major axis/minor axis is equal to or greater than 1.5 (YES), the chromosomal roundness is calculated in step S111, and this roundness is determined in step S112 as to whether it is equal to or greater than 0.5. When the roundness is not equal to or greater than 0.5 (NO), the stage of the cell is determined as metaphase in step S117. When the roundness is equal to or greater than 0.5 (YES), the stage of the cell is determined as prometaphase in step S118.

Following the determination of the stage of the cell in any one of the steps S113, S114, S115, S116, S117 and S118, the determination results are stored at the memory unit 42 so as to match to the cell ID Number in step S119, and then step S120 is executed. In the step S120, determination is made as to whether determination of all cell images was completed. If not completed (NO), the step S101 is executed back again, and the cell assigned with the next ID Number is subjected to the same processing as described in the foregoing. If the determination of all the cell images is completed (YES), the processing of the stage determination is terminated. In the step S107, the chromosomal roundness of the cell in the anaphase or telophase shall be calculated, therefore, two chromosomal images will be generally included in one cell image. The roundness in this step may be an average value of two chromosomal images, or may be a value decided based on the datum of one chromosomal image. Also in the step S101, the same will be applied when two chromosomal images are included in one cell image.

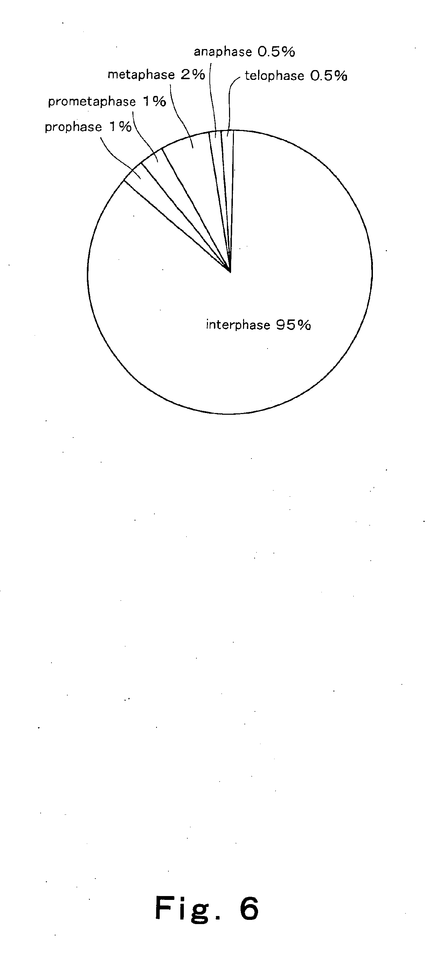

Referring back to Fig. 4, when the stage determination is terminated in the step S3, the determination results are displayed in step S4. When the monitor 5a is used as the output device 5, the determination results are displayed on a monitor as shown in the step S4, however, a printer is used as the output device 5, for example, the determination results are printed. Fig. 6 shows one example of a display of the determination results in the step S4. In Fig. 6, proportions of the cells in respective stages are represented on a circle graph. Since the stage determination of all the cells is completed in the step S3, proportion of cell number in each stage can be represented on a circle graph on the basis of the results of the stage determination as shown in Fig. 6, for example.

Next, in step S5, the mode set by the operator is discriminated whether it is a time-lapse mode or a count mode. When it is a count mode, the processing is terminated. To the contrary, when it is a time-lapse mode, lapse of the set tracking time period is determined in step S6. If not (NO), the step S1 is executed back again, and similar processing is carried out. The imaging in the step S1 is set so as to be executed every certain time period, for example, once per minute. When the lapse of the set time period is determined in the step S6 (YES), step S7 is then executed, and time-lapse analysis is carried out on all the cells. Thereafter, the cells after passage of all the steps for the mitotic phase are determined on the basis of the results of time-lapse analysis, and the results of time-lapse analysis suggesting the mitotic phase are displayed in step S8 on only these cells. Furthermore, in the step S8, average values of the results of time-lapse analysis of these cells are displayed.

In the processing shown in Fig. 4, when the count mode is employed, comparison of the determination results displayed in the step S4 with the data of the control cells enables the operator to visually determine extent of the subject activity (for example, whether or not the subject is active). In the image analysis apparatus 4, comparison with the control may be carried out automatically, and the extent of the activity may be determined automatically.

In the processing shown in Fig. 4, when the time-lapse mode is employed, comparison of the determination results displayed in the step S8 with the data of the control cells enables the operator to visually determine extent of the subject activity (for example, whether or not the subject is active). In the image analysis apparatus 4, comparison with the control may be carried out automatically, and the extent of the activity may be determined automatically. Also, in the time-lapse mode, determination of the degree of activity of the subject can be also carried out in a similar manner to the aforementioned count mode on the basis of the determination results displayed in the step S4.

Example in which the processing in the step S3 of the flow chart shown in Fig. 4 is different from the proceeding according to the flow chart shown in Fig. 5 will be explained below. In this Example, only the proceeding in the step S3 is different from the Example as described above, and the rests are similar. Therefore, explanation of the similar rests will be omitted.

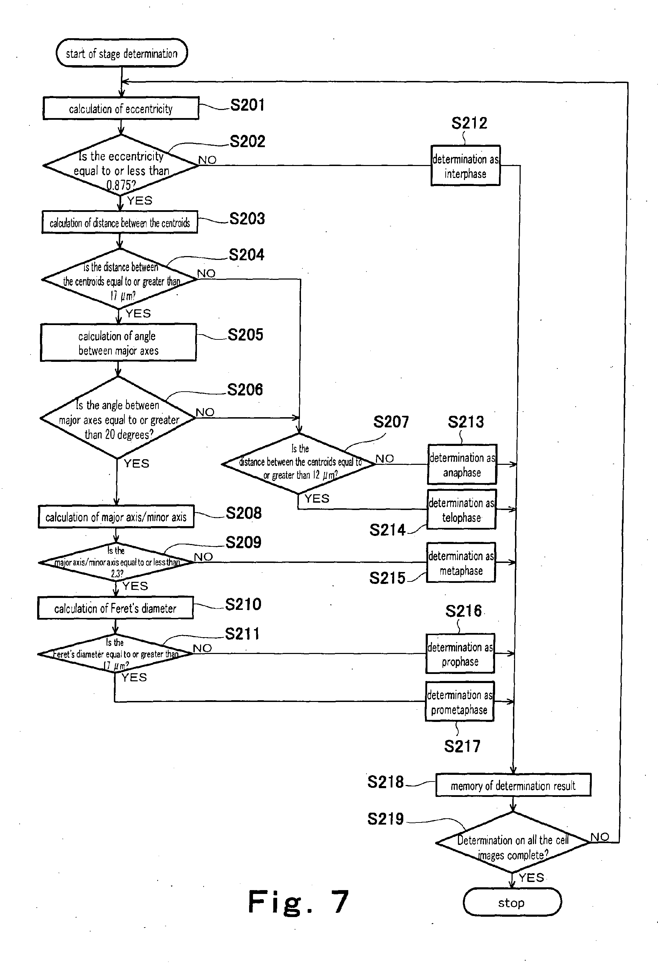

Fig. 7 shows a drawing illustrating specific processing procedures of stage determination of each cell in the step S3 of the flow chart shown in Fig. 4 in this Example. Fig. 7 shows controlling procedures when the monitoring subject cell is a HeLa cell. As shown in Fig. 7, upon initiation of the stage determination, the following processing is carried out with respect to the cell of ID No. 1 based on the cell image of ID No. 1. In step S201, eccentricity is calculated. Then, in step S202, the eccentricity is determined as to whether it is equal to or less than 0.875. If not (NO), the stage of the cell is determined as interphase in step S212. When the eccentricity is equal to or less than 0.875 (YES), the distance between the centroids of a chromosome and a chromosome adjacent thereto is calculated in step S203. Then, the distance between the centroids calculated in the step S203 is determined as to whether it is equal to or greater than 17 µm in step S204. If not (NO), step S207 is executed. To the contrary, when the distance between the centroids calculated in the step S203 is equal to or greater than 17 µm (YES), the angle formed by a chromosomal major axis and the major axis of a chromosome adjacent thereto is calculated in step S205. Then, the angle calculated in the step S205 is determined as to whether it is equal to or greater than 20 degrees in step S206. If not (NO), step S207 is executed. To the contrary, when the angle calculated in the step S205 is equal to or greater than 20 degrees (YES), step S208 is executed.

In the step S207, the distance between the centroids calculated in the step S203 is determined as to whether it is equal to or greater than 12 µm. When the distance between the centroids is equal to or greater than 12 µm (YES), the stage of the cell is determined as telophase in step S214. When the distance between the centroids in mitotic phase is not equal to or greater than 12 µm (NO), the stage of the cell is determined as anaphase in step S213. In the step S208, the ratio of the major axis to the minor axis of the chromosome (major axis/minor axis) is calculated, and in step S209, the major axis/minor axis is determined as to whether it is equal to or less than 2.3. If not (NO), the stage of the cell is determined as metaphase in step S215. When the major axis/minor axis is equal to or less than 2.3 (YES), the chromosomal Feret's diameter is calculated in step S210, and the Feret's diameter is determined as to whether it is equal to or greater than 17 µm in step S211. When the Feret's diameter is not equal to or greater than 17 µm (NO) the stage of the cell is determined as prophase in step S216. When the Feret's diameter is equal to or greater than 17 µm (YES), the stage of the cell is determined as prometaphase in step S217. Following the determination of the stage of the cell in any one of the steps S212, S213, S214, S215, S216 and S217, the determination results are stored at the memory unit 42 so as to match to the cell ID Number in step S218, and then step S219 is executed. In the step S219, determination is made as to whether determination of all cell images was completed. If not completed (NO), the step S201 is executed back again, and the cell assigned with the next ID Number is subjected to the same processing as described in the foregoing. If the determination of all the cell images is completed (YES), the processing of the stage determination is terminated. In the step S201, it happens that two chromosomal images are included in one cell image. In this case, either an average value of the two chromosomal images, or a value decided based on the datum of one chromosomal image is acceptable.

### Experiments

### 1. Construction of GFP-histone H1-expressing HeLa cell

Chromosomes are constructed from DNAs and chromosomal proteins. In the above Example, histone H1.2 was selected which is constitutively expressed in almost all tissues (Meergans et al., 1997) in order to achieve visualization of the chromosomes in living cells through fusion of histone H1 that is a main chromosomal protein with GFP that is a fluorescence protein.

A histone H1.2 gene was obtained from a human genome by PCR, and subcloned into pUC18. Thereafter, following cleavage with a restriction enzymatic treatment, cloning into pEGFP-Cl (Clontech) which is a GFP fusion protein expression vector in animal cells was carried out.

Thus constructed vector was introduced into HeLa cells with lipofectamine (Invitrogen Corporation). Transformant was selected by an antibiotic G418 (final concentration: 800 mg/mL). Thus selected clone was confirmed to express the fusion protein in all cells even after subjecting to passage culture four times (diluted to 1/10 concentration each time), therefore, it was decided as a clone that was constitutively expressing the GFP-histone H1 fusion protein. Moreover, this clone was confirmed to keep the ability to express the fusion gene even after carrying out cryopreservation and thawing in liquid nitrogen.

### 2. Evaluation of Cell Activity by Identification Method of Chromosome Structural Alteration

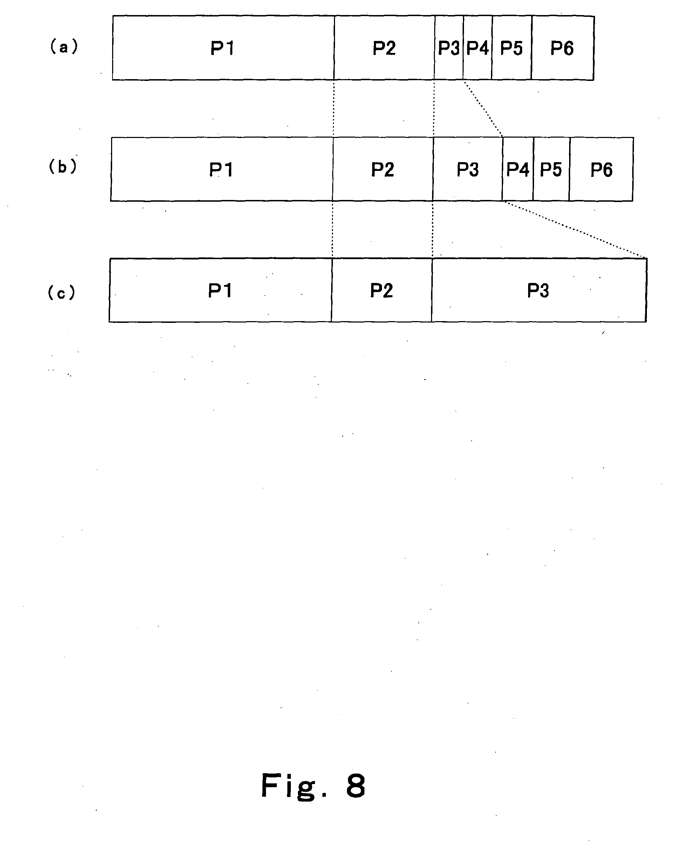

With respect to the GFP-histone H1-expressing HeLa cell, determination with a time-lapse mode was carried out by the system in which the flow chart shown in Fig. 7 was employed in the stage determination of the step S3 shown in Fig. 4 among the systems for cell-based assay as described above. Fig. 8 illustrates average values in the step S8 shown in Fig. 4 in terms of the results of the time-lapse analysis. Fig. 8 (a) shows the results of the control time-lapse analysis; Fig. 8 (b) shows the results of the time-lapse analysis of the subject to which a metaphase inhibitor, nocodazole, was added at a final concentration of 15 ng/ml; and Fig. 8 (c) shows the results of the time-lapse analysis of the subject to which a metaphase inhibitor, nocodazole, was added at a final concentration of 150 ng/m. In Figs. 8 (a) to (c), the abscissa represents time; P1 represents the interphase; P2 represents the prophase; P3 represents the prometaphase; P4 represents the metaphase; P5 represents the anaphase; and P6 represents the telophase. From Fig. 8 (c), it is revealed that when the metaphase inhibitor, nocodazole, was added at a final concentration of 150 ng/ml, cell division was arrested at the prometaphase. Moreover, Fig. 8 (b) also reveals that the addition of the metaphase inhibitor, nocodazole, at a final concentration of 15 ng/ml prolonged the prometaphase. Accordingly, lowering of the cell activity can be grasped by way of alteration of the time period in a particular stage of the cell division. Hence, it is suggested that monitoring of influences of an inhibitory substance upon a cell activity is enabled at a low concentration of the substance which could not be detected by conventional methods of evaluating the cell activity on fixed cells.

### INDUSTRIAL APPLICABILITY

The method for monitoring a cell, the system for cell-based assay, and the program for cell-based assay according to the present invention are useful for poison tests, environmental endocrine disrupter tests, medicament responsiveness tests, and the like.

## Claims

1. A method for monitoring a cell comprising:
(a) obtaining an image that reflects the chromosomal state in the cell; and
(b) calculating a parameter that corresponds to the chromosomal state based on the image to determine the stage in the cell cycle on the basis of the calculation result.

2. The method for monitoring a cell according to claim 1 wherein the parameter comprises chromosomal circularity, chromosomal roundness, ratio of lengths of chromosomal major axis and minor axis, chromosomal eccentricity, chromosomal Feret's diameter, distance between the centroids of a chromosome and a chromosome adjacent thereto, angle formed by a chromosomal major axis and the major axis of a chromosome adjacent thereto, or any combination thereof.

3. The method for monitoring a cell according to claim 1 or 2 which further comprises:
(c) allowing a fusion protein of a chromosomal protein and a protein that generates fluorescence to be expressed in the cell before the step (a), wherein
the image is a fluorescence image originating from the fluorescence generated by the cell in the step (a).

4. The method for monitoring a cell according to any one of claims 1 to 3 which further comprises:
(d) evaluating the degree of activity of the cell on the basis of the result of determination in the step (b).

5. The method for monitoring a cell according to claim 4 wherein:
the images that correspond to multiple cells in a sample are obtained in the step (a);
the stages are determined respectively on the multiple cells in the step (b); and
the proportion of cell number in each stage is calculated in the step (d), whereby the degree of activity of the cell is evaluated on the basis of the calculation result.

6. The method for monitoring a cell according to claim 4 wherein
the images that correspond to a particular cell are obtained at certain time intervals in the step (a);
the stage at certain time intervals of the particular cell is determined in the step (b); and
the degree of activity of the cell is evaluated on the basis of the information of alteration of the stage of the particular cell with respect to time course in the step (d).

7. A system for cell-based assay comprising: a fluorescence detection apparatus for detecting fluorescence that reflects the chromosomal state in a cell; an imaging apparatus for obtaining a fluorescence image originating from fluorescence detected by the fluorescence detection apparatus; and an image analysis apparatus for calculating a parameter that corresponds to the chromosomal state based on the fluorescence image obtained by the imaging apparatus, and determining the stage in the cell cycle on the basis of the calculation result.

8. The system for cell-based assay according to claim 7 wherein
the fluorescence detection apparatus simultaneously detects fluorescence derived from multiple cells in a sample;
the imaging apparatus obtains the fluorescence image involving the fluorescent information generated by the multiple cells in the sample; and
the image analysis apparatus extracts the fluorescence image that corresponds to each cell from the fluorescence image obtained by the imaging apparatus to determine the stage in cell cycle on each cell.

9. The system for cell-based assay according to claim 7 wherein
the imaging apparatus obtains the fluorescence image that corresponds to a particular cell at certain time intervals, and
the image analysis apparatus determines the stage of the particular cell at the certain time intervals.

10. A program for cell-based assay wherein: fluorescence that reflects the chromosomal state in a cell is detected; a fluorescence image is obtained from the fluorescence; a parameter that corresponds to the chromosomal state is calculated based on the fluorescence image; and a computer is allowed to execute procedures for determining the stage in the cell cycle on the basis of the calculation result.

11. A computer readable recording medium in which a program for cell-based assay is recorded wherein:
fluorescence that reflects the chromosomal state in a cell is detected; a fluorescence image is obtained from the fluorescence; a parameter that corresponds to the chromosomal state is calculated based on the fluorescence
image; and a computer is allowed to execute procedures for determining the stage in the cell cycle on the basis of the calculation result.
